# EUROPEAN PATENT APPLICATION

(11) **EP 4 702 999 A1**
(43) Date of publication of application: **04.03.2026**
(21) Application number: 24197429.4
(22) Date of filing: 29.08.2024
(51) Int. Cl.: A61M 11/06, A61M 13/00, A61B 17/00

(54) **MEDICAL POWDER SPRAY APPARATUS**

(71) Applicant: Theracion Biomedical Co. Ltd., Seongnam-si 13202 (KR)
(72) Inventor: Kim, Eun Jin, 13587 Seongnam-si, Gyeonggi-do (KR); Jeon, Soo Hyeon, 12773 Gwangju-si, Gyeonggi-do (KR); Jo, Hee Jung, 13154 Seongnam-si, Gyeonggi-do (KR); Kim, Byung Nam, 13205 Seongnam-si, Gyeonggi-do (KR)
(74) Representative: Ter Meer Steinmeister & Partner

(57) **Abstract**

Proposed is a medical powder spray apparatus, including a powder accommodation part (10) accommodating powder, a powder discharge means (20) discharging the powder of the powder accommodation part (10), a powder transfer pipe (30) transferring the powder of the powder discharge means (20), an air transfer pipe (40) transferring air together with the powder of the powder transfer pipe (30), an air supply part (50) supplying the air to the air transfer pipe (40), and a spray means (50) spraying the powder of the air transfer pipe (40). The powder is sprayed by means of wind pressure.

## Description

### BACKGROUND OF THE INVENTION

### Field of the Invention

The present disclosure relates to a medical powder spray apparatus that can spray powder-type medications in an accurate and fixed amount for various medical purposes.

### Description of the Related Art

Surgery refers to a medical practice in which a person's skin or mucous membranes are incised with medical instruments and body parts causing problems in sustaining life are treated using medical technology. These surgeries are classified as medical or surgical according to body parts. Depending on the severity of surgery, there are cases where a patient's life is at risk. Additionally, there is a risk that various unexpected accidents, such as bleeding and adhesion, may occur during surgery. In an effort to prevent this or resolve it when it occurs, various medications, such as hemostatic agents, anti-adhesion agents, and wound dressings, have been developed to improve the success rate of surgery and patient survival rate.

For example, bleeding means that blood leaks from blood vessels. The blood vessels circulate blood inside the blood vessels throughout the whole body from the heart by the pressure generated by the heart. When a wound occurs in some blood vessels, an opening is created, and blood leak outs of the wound due to the pressure.

Hemostasis refers to the process of stopping bleeding and keeping blood within damaged blood vessels. During hemostasis, it is critically important to quickly stop bleeding from a bleeding site by suturing or compressing a wound area with a hemostatic agent, bandage, or dressing. In particular, recently, the demand for hemostatic agents that are human-friendly, have excellent biocompatibility, and have excellent hemostatic properties has been increasing. In response to this, hemostatic agents using non-toxic, biocompatible polymers as main materials are under development to meet improved hemostatic effects and various market demands.

After tissue is damaged by bleeding, wounds, friction, etc. due to surgery or other reasons, blood coagulates during the healing of the wound. Tissue adhesion refers to a phenomenon in which abnormal adhesion with surrounding tissues occur during the coagulating of blood. Therefore, it is common to use anti-adhesion agents to prevent this.

Additionally, a wound refers to a case in which the continuity of human tissue is destroyed by external pressure or other factors. During treatment, a wound has a risk of being infected or inflamed when exposed to the external environment. To prevent this, medications such as wound dressing agents are manufactured and used.

These medications have been developed into various shapes, such as hydrogel, sponge, matrix, powder, sheet, etc., optimized for use or surgical methods. In particular, powder-type medications are easier to apply evenly to curved human tissue than other forms, and are advantageous for treating large areas.

However, even when a hemostatic agent, anti-adhesion agent, or wound dressing agent is developed for this purpose, it is also essential to apply or dose these agents in an appropriate amount at a target location. For example, a nozzle or tube for applying medications may vary in material, length, and diameter depending on various purposes such as surgical operation, procedure, laparoscopy, and endoscopy, so the nozzle for applying these medications needs to be replaced frequently to be suitable for the purpose as necessary. Such replacement is required to be made quickly and simply.

While researching to solve the above problems, the inventors of the present application have proposed a multi-purpose medical powder spray device that allows a nozzle part to be replaced depending on the body part or purpose to which medical powder is sprayed, allowing an appropriate amount of medical powder to be sprayed at a target location, and filed for a patent (Korean Patent Application No. 10-2020-0027493).

Meanwhile, in recent years, polypectomy of the gastrointestinal or colon and mucosal resection for the treatment of early gastric cancer and colorectal cancer, and therapeutic endoscopic procedures have been widely performed. Such procedures are accompanied by a risk of bleeding during or after the procedures, so in severe cases, emergency surgery may be required or even death may occur. Therefore, hemostasis using an endoscope has been increasingly used. Hemostasis using the endoscope is a method of stopping bleeding by accessing an endoscope conduit (catheter) inserted into the body to a damaged mucous membrane and then spraying and dosing a powder-type hemostatic agent though the conduit.

Conventional spray devices that deliver medications using air pressure generated by manually applying compression force are insufficient in performance to pass through long endoscope conduits. Recently, spray devices that can pass through an endoscope conduit using strong air pressure and continuous air supply generated by motorized devices or gas generators have been developed.

Accordingly, the inventors of the present application have improved the above-described current spray devices and proposed a spray device that uses a motorized device to allow medications to pass through an endoscope conduit and easily adjust a spray amount, and filed for a patent (Korean Patent Application No. 10-2020-0117158).

Meanwhile, such a conventional spray device controls spraying by simply blocking or opening/closing a spray port, making it difficult to finely adjust the spray amount as needed by users, and a flow of air inside a sprayer is not uniform, making it difficult to deliver medications evenly.

While further researching to solve the above problems, the inventors of the present application have completed the present disclosure by discovering that when using a separate flow control device and a passage device for adjusting the wind direction, it is possible to finely adjust the spray amount according to user's needs and improve the stability of the spray device by uniformly delivering medications and preventing clogging inside the sprayer.

The foregoing is intended merely to aid in the understanding of the background of the present disclosure, and is not intended to mean that the present disclosure falls within the purview of the related art that is already known to those skilled in the art.

### SUMMARY OF THE INVENTION

Accordingly, the present disclosure has been made keeping in mind the above problems occurring in the related art, and the present disclosure provides a medical powder spray apparatus having a flow rate control means.

In particular, the present disclosure provides a medical powder spray apparatus that can smoothly supply powder through vibration and prevent residual or backflow of powder by maintaining a uniform air flow for transferring the powder.

In order to achieve the above objectives, according to one aspect of the present disclosure, there is provided a medical powder spray apparatus, including: a powder accommodation part accommodating powder; a powder discharge means guiding and discharging the powder accommodated in the powder accommodation part out of the powder accommodation part; a powder transfer pipe transferring the powder discharged from the powder discharge means; an air transfer pipe transferring air together with the powder supplied from the powder transfer pipe; an air supply part supplying the air to the air transfer pipe; a spray means spraying the powder transferred to the air transfer pipe by means of the air from the air supply part to outside; and a controller controlling operation of the powder discharge means or the air supply part by supplying power to at least one of the powder discharge means and the air supply part.

The air supply part may supply the air to the air transfer pipe to push the powder transferred to the air transfer pipe to the spray means.

The controller may include: a power supply part supplying the power to at least one of the powder discharge means and the air supply part; and a trigger part switched to apply the power of the power supply part to at least one of the powder discharge means and the air supply part.

The powder discharge means may include: a vibration generation part generating vibration; a vibration transmission part transmitting the vibration from the vibration generation part to the powder accommodation part; and a powder discharge part dispersing and discharging the powder in the powder accommodation part while being fixed to the vibration transmission part.

The vibration transmission part may include: a vibration pipe being in contact with the vibration generation part, communicating with the powder transfer pipe, and allowing the powder to move therethrough; and a funnel formed at an end of the vibration pipe, receiving the powder discharge part, and fixed in a state in communication with the powder accommodation part.

A support step into which the powder discharge part is insertedly fitted and supported may be formed on an inner side of the funnel.

The powder discharge part may be formed in the shape of a triangular pyramid, a quadrangular pyramid, or a cone, and may have a plurality of holes allowing the powder to pass therethrough in a dispersed state.

The holes of the powder discharge part may be formed to gradually become larger from an upper portion to a lower portion of the powder discharge part.

A backflow prevention part preventing backflow of the powder may be formed in the air transfer pipe.

The backflow prevention part may include: a ring body installed in the air transfer pipe and having a space that allows the air to pass therethrough; and a pair of prevention barrier formed on opposite sides of the ring body, respectively, and opened when the air is supplied from the air transfer pipe and closed when the air is not supplied from the air transfer pipe.

Each of the prevention barrier may be made of a flexible rubber plate having a half-disk shape, and the prevention barrier may be formed such that at least a portion of a semicircular edge of each of the prevention barrier is fixed to an inner side of the ring body and straight edges of the prevention barrier are in contact with each other while facing each other.

An air flow maintenance part maintaining a flow of the air at a uniform pressure may be mounted in the air transfer pipe.

The air flow maintenance part may be configured as a screw that forms a vortex of the air moving in the air transfer pipe, or a pipe having a plurality of holes dispersing the air moving in the air transfer pipe.

The trigger part may include: a trigger manipulated with a finger; and a switch operated in response to manipulation of the trigger and switched to transmit the power of the power supply part to the powder discharge means or the air supply part.

The air supply part may be configured to adjust wind pressure of the air, so that the air supply part may supply the air at low pressure in response to application of low voltage power and supply the air at high pressure in response to application of high voltage power.

The controller may include: a transformer part changing voltage of the power supplied from the power supply part to the air supply part to adjust the wind pressure of the air supplied from the air transfer pipe; and a voltage change display part displaying a voltage change state of the transformer part.

The transformer part may be controlled in operation by any one of the trigger part and the voltage change manipulation part operated for voltage change.

The voltage change display part may be configured as a plurality of LEDs. The LEDs may be set such that a small number of LEDs are turned on at low voltage and a large number of LEDs are turned on at high voltage, and the LEDs may emit different colors depending on a voltage level.

According to the present disclosure, by spraying medical powder medications corresponding to various types of medical procedures through air of the air supply part, it is possible to spray the powder medications in an accurate and fixed amount.

Additionally, by discharging powder through vibration, it is possible to smoothly provide the powder, and when the user generates vibration while the powder accommodated in the powder accommodation part does not pass through the powder discharge part, it is possible to enable the powder to easily pass through the powder discharge part.

In addition, by providing the powder discharge part having a cone shape and having a plurality of holes, it is possible to enable the powder to pass through the entire powder discharge part evenly and be supplied in a fixed amount.

Additionally, by forming the holes of the powder discharge part to gradually become larger from the upper to lower portion of the powder discharge part, it is possible to enable the powder to be smoothly discharged from the lower portion of the powder discharge part.

Additionally, by preventing backflow of powder through the backflow prevention part formed in the air transfer pipe, it is possible to prevent the powder from remaining in the air transfer pipe and to prevent apparatus failure caused by the powder.

Additionally, by providing the air flow maintenance part mounted in the air transfer pipe, it possible to enable a flow of air to be maintained at a uniform pressure, thereby evenly spraying the powder and preventing the powder from remaining in the air transfer pipe.

Additionally, by enabling the trigger to manipulate the switch that applies power, it is possible to operate the switch accurately.

Additionally, by supplying air at low or high pressure through the air transfer pipe, it is possible to adjust the spray intensity of the powder.

### BRIEF DESCRIPTION OF THE DRAWINGS

The above and other objectives, features, and other advantages of the present disclosure will be more clearly understood from the following detailed description when taken in conjunction with the accompanying drawings, in which:
FIG. 1 is a perspective view illustrating a medical powder spray apparatus according to an embodiment of the present disclosure;
FIG. 2 is an exploded perspective view illustrating a powder discharge means according to the present disclosure;
FIG. 3 is a perspective view illustrating an assembled state of FIG. 2;
FIG. 4 is an exploded perspective view illustrating a backflow prevention part according to the present disclosure;
FIGS. 5A and 5B are sectional views illustrating an embodiment of an air flow maintenance part according to the present disclosure; and
FIGS. 6A and 6B are perspective views illustrating another embodiment of an air flow maintenance part according to the present disclosure.

### DETAILED DESCRIPTION OF THE INVENTION

Hereinbelow, an exemplary embodiment of the present disclosure will be described in detail with reference to the accompanying drawings.

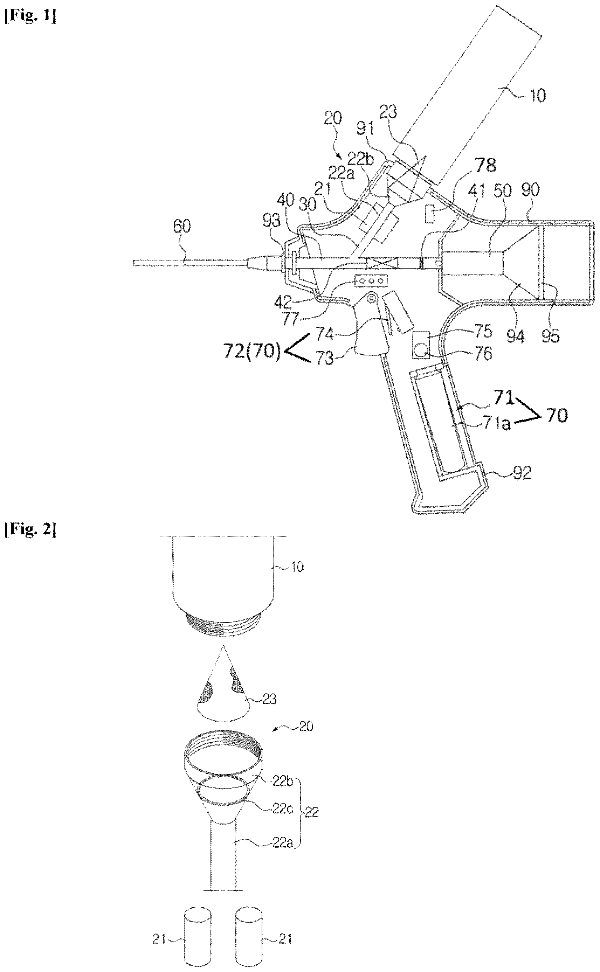

As illustrated in FIG. 1, a medical powder spray apparatus according to an embodiment of the present disclosure includes a powder accommodation part 10, a powder discharge means 20, a powder transfer pipe 30, an air transfer pipe 40, an air supply part 50, and a spray means 60.

First, these configurations are mounted on a pistol-shaped body 90 so that a user can conveniently hold and use them by hand.

As illustrated in FIGS. 1 to 4, the powder accommodation part 10 may be configured as a container to accommodate powder. The container may have a threaded opening and be formed transparent or translucent so that the interior thereof is visible.

A powder mounting port 91 in which the powder accommodation part 10 is mounted is formed at an upper portion of the body 90. The powder mounting port 91 is formed in a cylindrical shape so that the opening of the powder accommodation part 10 is inserted and mounted therein. The powder mounting port 91 communicates with the powder transfer pipe 30 and has threads formed on an inner circumferential surface thereof so that the inlet of the powder accommodation part 10 is screwed thereto.

The powder discharge means 20 functions to guide and discharge powder filled in the powder accommodation part 10 out of the powder accommodation part 10 or to discharge an adjusted amount of powder from the powder accommodation part 10, and includes a vibration generation part 21 for generating vibration, a vibration transmission part 22 for transmitting the vibration from the vibration generation part 21 to the powder accommodation part 10, a powder discharge part 23 for dispersing and discharging the powder in the powder accommodation part 10 while being fixed to the vibration transmission part 22.

The vibration generation part 21 is configured as a general vibrator that operates when power is input and generates vibration, and is disposed in close contact with the vibration transmission part 22. Additionally, a plurality of vibrators may be provided and placed on opposite sides of the vibration transmission part 22. Due to this condition, the vibration generated in the vibration generation part 21 is easily transmitted to a vibration pipe 22a of the vibration transmission part 22. Therefore, this vibration is transferred to the powder discharge part 23, so blockage of the powder is resolved by the vibration. As a result, the powder is smoothly discharged through the powder discharge part 23.

The vibration transmission part 22 includes the vibration pipe 22a being in contact with the vibration generation part 21, communicating with the powder transfer pipe 30, and allowing the powder to move therethrough; and a funnel 22b formed at an end of the vibration pipe 22a, receiving the powder discharge part 23, and fixed in a state in communication with the powder accommodation part 10.

An upper portion of the funnel 22b is formed to be larger than a lower portion of the powder discharge part 23 to receive the lower portion of the powder discharge part 23.

A support step 22c into which the powder discharge part 23 is insertedly fitted and supported is formed on an inner side of the funnel 22b. The support step 22c is configured as a plurality of protrusions or is formed in a ring shape. When the powder discharge part 23 is supported on the support step 22c, the powder discharge part 23 maintains an upright posture without being tilted to one side inside the funnel 22b.

The powder discharge part 23 is formed in the shape of a triangular pyramid, a quadrangular pyramid, or a cone, and has a plurality of holes formed dispersedly and allowing the powder to pass therethrough. When vibration is transmitted to the powder discharge part 23 in a state where the powder in the powder accommodation part 10 clogs in the holes and cannot pass through them, the powder is caused to pass through the holes by the vibration.

Here, when a large amount of powder falls from the powder accommodation part 10 simultaneously due to gravity, the vibration pipe 22a or the powder transfer pipe 30 may be clogged. However, the vibration pipe 22a or the powder transfer pipe 30 is not clogged by the powder because the powder is not discharged simultaneously through the holes formed densely in the powder discharge part 23, but is dispersed and discharged in small amounts.

The holes of the powder discharge part 23 may be formed in various shapes such as a triangle, a quadrangle, a circle, a pentagon, and a hexagon by perforating, but the shapes thereof are not limited thereto.

Additionally, the holes of the powder discharge part 23 may be formed to have the same size or may be formed to gradually become larger from the upper portion to the lower portion of the powder discharge part. When the holes of the powder discharge part 23 gradually become larger from the upper portion to the lower portion of the powder discharge part, the powder can be smoothly discharged through the lower portion of the powder discharge part 23.

Here, the holes may have a diameter of 5 to 600 micrometers. More preferably, the holes may have a diameter of 5 to 200 micrometers. Even more preferably, the holes may have a diameter of 50 to 200 micrometers. Of course, the diameter of the holes may be appropriately controlled depending on the size of the powder. That is, the holes need to be formed larger than the size of the powder, so the diameter thereof is determined by the size of the powder.

When the vibration generation part 21 is vibrated as described above, the vibration pipe 22a and the funnel 22b are vibrated together with the powder discharge part 23. The powder in the powder accommodation part 10 is dispersed and discharged through the powder discharge part 23 by vibration. The powder discharged through the powder discharge part 23 is collected through the funnel 22b and then supplied to the powder transfer pipe 30 through the vibration pipe 22a. The powder supplied to the powder transfer pipe 30 is supplied to the air transfer pipe 40.

The powder transfer pipe 30 functions to transfer the powder discharged from the powder discharge means 20, and is connected to the air transfer pipe 40 in an inclined state. The powder flows through the inclined powder transfer pipe 30 and is supplied to the air transfer pipe 40.

The air transfer pipe 40 functions to transfer air together with the powder supplied from the powder transfer pipe 30. The air transfer pipe 40 has an end located at a front end of the body 90.

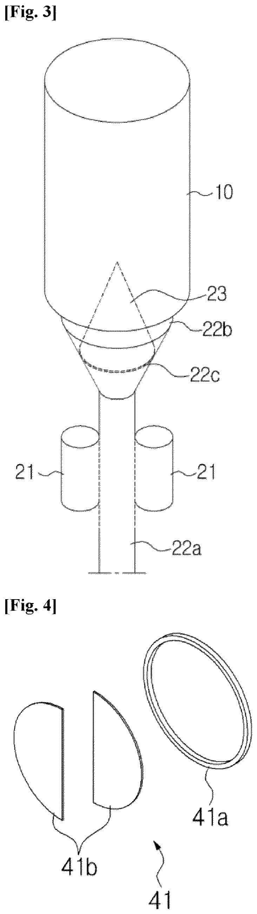

As illustrated in FIG. 1 and FIG. 4, a backflow prevention part 41 for preventing backflow of the powder is formed in the air transfer pipe 40.

The backflow prevention part 41 includes a ring body 41a installed in the air transfer pipe 40 and having a space that allows the air to pass therethrough; and a pair of prevention barrier 41b formed on opposite sides of the ring body 41a, respectively, and opened when the air is supplied from the air transfer pipe 40 and closed when the air is not supplied from the air transfer pipe 40.

The ring body 41a is installed so that an outer peripheral edge thereof is in close contact with an inner peripheral surface of the air transfer pipe 40 to prevent the air from leaking through the rim.

Each of the prevention barrier 41b is configured as a diaphragm in which a flexible rubber plate is formed in a half-disk shape. The prevention barrier 41b are formed such that at least a portion of a semicircular edge of each of the prevention barrier 41b is fixed to an inner side of the ring body 41a and straight edges of the prevention barrier 41b are in contact with each other while facing each other. It is preferable that each of the prevention barrier 41b is fixed to an inner peripheral edge of the ring body 41a only at a center portion of a semicircular edge thereof. Therefore, the prevention barrier 41b open and close an inner peripheral surface of the ring body 41a while fluttering around fixed portions thereof.

With this configuration, when the air is supplied from the air transfer pipe 40, the prevention barrier 41b are opened with ends thereof facing away from each other with a gap therebetween to allow the air to pass through the gap. When the supply of air from the air transfer pipe 40 is interrupted, the ends of the prevention barrier 41b are brought into contact with each other while facing each other and are closed. Therefore, the powder cannot flow back after passing through the prevention barrier 41b.

As a result, since the powder is prevented from flowing back due to the backflow prevention part 41, the powder does not remain inside the air transfer pipe 40. This prevents the apparatus from breaking down due to remaining powder.

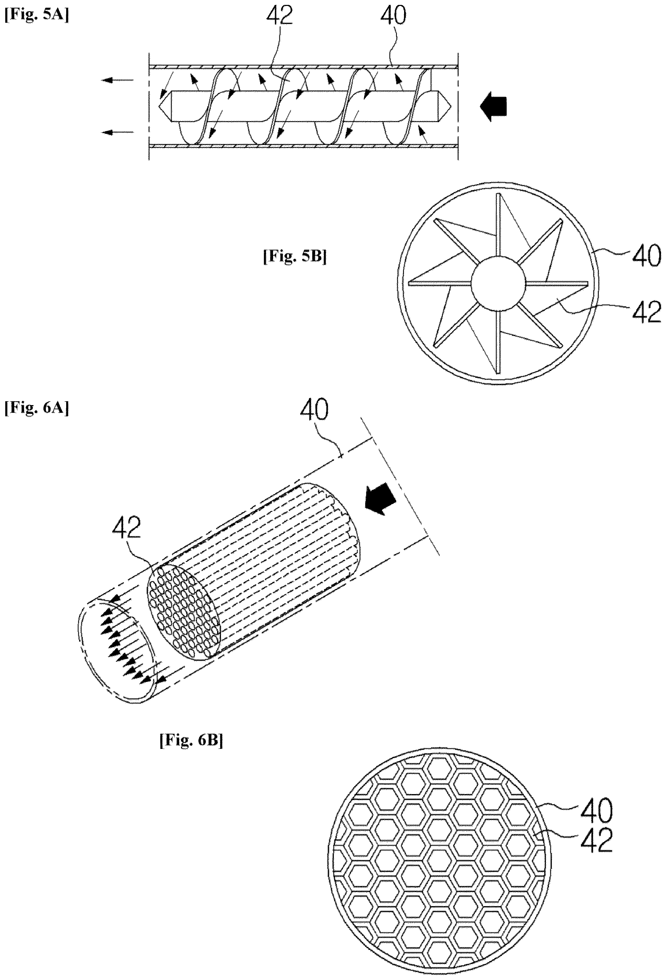

As illustrated in FIG. 1 and FIGS. 5A and 5B, an air flow maintenance part 42for maintaining a flow of the air at a uniform pressure is mounted in the air transfer pipe 40.

The air flow maintenance part 42 may be configured as a screw that forms a vortex of the air moving in the air transfer pipe 40. The screw includes blades that are formed in a spiral shape around a central shaft. The central shaft is located at the center of the air transfer pipe 40, and the spiral blades are in close contact with the inner peripheral surface of the air transfer pipe 40. Therefore, the air transferred from the air transfer pipe 40 forms a vortex as it moves along the spiral blades. The air that forms this vortex is prevented from being biased by the vortex or transfers the powder while flowing at a uniform pressure. Therefore, the powder can be sprayed evenly and does not remain in the air transfer pipe 40.

As illustrated in FIG. 1 and FIGS. 6A and 6B, the air flow maintenance part 42 may be configured as a pipe having a plurality of holes for dispersing the air moving in the air transfer pipe 40. This pipe is formed such that the plurality of holes are arranged densely together to form a honeycomb shape. Therefore, the air transferred from the air transfer pipe 40 is dispersed while passing through the honeycomb-shaped holes, so it is prevented from being biased or flows at a uniform pressure.

As described above, the air transferred at a uniform pressure by the air flow maintenance part 42 transfers the powder by moving evenly throughout the inside of the air transfer pipe 40 without being biased to one side. As a result, the powder is supplied to the spray means 60 without remaining accumulated in the lower portion of the air transfer pipe 40.

As illustrated in FIG. 1, the air supply part 50 functions to supply the air to the air transfer pipe 40 and may be configured as an air pump or motor. The air pump supplies the air by pumping, and the motor supplies the air by increasing the number of revolutions.

The air supply part 50 communicates with the air inlet 94 formed at a rear end of the body 90 while being connected to the air transfer pipe 40. Therefore, when the air supply part 50 is operated, the air is introduced through the air inlet 94 and is supplied to the air transfer pipe 40.

Here, the air inlet 94 is provided with an air filter 95 for filtering out dust from the introduced air. That is, the air transfer pipe 40 can provide clean air from which dust has been removed.

The spray means 60 functions to spray the air together with the powder transferred from the air transfer pipe 40 and may be in the form of a tube or needle.

The spray means 60 is connected to a spray connection part 93 formed at the front end of the body 90. The spray connection part 93 may be configured as a force fit type in which the spray means 60 is forcibly inserted and connected thereto, or a luer lock type in which the spray means 60 is connected thereto in a screwing manner.

Here, the above-described air supply part 50 is set to supply the air at low pressure in response to application of low voltage power and to supply the air at high pressure in response to application of high voltage power.

Additionally, the present disclosure further includes a controller 70 for controlling operation of the powder discharge means 20 and/or the air supply part 50 by supplying power thereto.

The controller 70 includes a power supply part 71 for supplying power to at least one of the powder discharge means 20 and the air supply part 50, and a trigger part 72 for applying the power of the power supply part 71 to the powder discharge means 20 and/or the air supply part 50.

The power supply part 71 functions to supply power to the powder discharge means 20 and/or the air supply part 50, and includes a battery 71a for supply charging power to the powder discharge means 20 and/or the air supply part 50 and a constant voltage regulator 78 for adjusting the power supplied from the battery 71a to the powder discharge means 20 to a constant voltage.

Therefore, the power supply part 71 supplies power to the powder discharge means 20 and the air supply part 50 in response to the operation of the trigger part 72. The powder supplied through the powder discharge means 20 is supplied to the spray means 60 by the air from the air supply part 50 operated by power.

The trigger part 72 functions to operate the powder discharge means 20 and the air supply part 50, and includes a trigger 73 manipulated with a finger and a switch 74 operated in response to the manipulation of the trigger 73 and switched to transmit the power of the power supply part 71 to the powder discharge means 20 and the air supply part 50.

The trigger 73 is formed to protrude outward from a handle 92 formed at the lower portion of the body 90. The user may manipulate the trigger 73 with his or her finger while holding the handle 92.

The switch 74 is electrically connected to the powder discharge means 20 and/or the air supply part 50 by the power supply part 71. When the switch 74 is manipulated, the power from the power supply part 71 is supplied to the powder discharge means 20 and/or the air supply part 50.

Therefore, when the user manipulates the trigger part 72 while holding the pistol-shaped body 90 with his or her hand, the switch 74 is switched to operate the powder discharge means 20 and/or the air supply part 50, causing the powder to be sprayed together with the air.

Here, since the trigger part 72 is operated in a manner that the switch 74 that applies power is operated through the trigger 73, the switch 74 can be switched reliably.

Additionally, in an embodiment of the present disclosure, when the switch 74 is operated, the air supply part 50 may be operated first and the powder discharge means 20 may be operated thereafter. Therefore, the powder is supplied to the powder transfer pipe 30 by the powder discharge means 20 while the air is constantly supplied by the air supply part 50, so the powder can be immediately sprayed without accumulating in the air transfer pipe 40.

In an embodiment of the present disclosure, when the operation of the switch 74 is stopped, the powder discharge means 20 may be stopped first and the air supply part 50 may be stopped thereafter. Therefore, the air is supplied by the air supply part 50 for a predetermined period of time even after the powder supply from the powder discharge means 20 is stopped, so the powder can be sprayed without remaining in the air transfer pipe 40.

Here, the above-described air supply part 50 supplies the air at low pressure in response to application of low voltage power and supplies the air at high pressure in response to application of high voltage power. That is, the air supply part 50 may adjust the wind pressure by adjusting the number of pumping strokes of the air pump or the number of revolutions of the motor according to voltage.

Additionally, the controller 70 includes a transformer part 75 for changing voltage of the power supplied from the power supply part 71 to the air supply part 50 to adjust the wind pressure of the air supplied from the air transfer pipe 40, a voltage change manipulation part 76 manipulated for voltage change, and a voltage change display part 77 for displaying a voltage change state in response to the manipulation of the voltage change manipulation part 76.

The transformer part 75 changes the voltage of the power supply part 71 by reducing or increasing the voltage. The transformer part 75 is set to transform the voltage in multiple steps, as will be described later. That is, the transformer part 75 changes the voltage to a preset voltage step by step according to a selected step.

The voltage change manipulation part 76 is configured as a button and is manipulated by the user. When the user manipulates the button of the voltage change manipulation part 76, the transformer part 75 is operated to reduce or increase the voltage of the power supplied from the power supply part 71.

The voltage change display part 77 is configured as a plurality of LEDs. The LEDs may be set such that a small number of LEDs are turned on at low voltage and a large number of LEDs are turned on at high voltage.

Therefore, when the user manipulates the button of the voltage change manipulation part 76 so that low voltage power is supplied to the air supply part 50, the transformer part 75 changes the voltage of the power supplied from the power supply part 71 to a low level. At this time, the LEDs of the voltage change display part 77 are turned on in small numbers.

When the user manipulates the button of the voltage change manipulation part 76 so that high voltage power is supplied to the air supply part 50, the transformer part 75 changes the voltage of the power supplied from the power supply part 71 to a high level. At this time, the LEDs of the voltage change display part 77 are turned on in increasing numbers. The voltage change display part 77 may be configured to be turned on in different colors depending on the changed voltage of the transformer part 75. For example, blue, yellow, and red colors may be turned on sequentially.

Therefore, the air supply part 50 may adjust the amount of powder sprayed through the air transfer pipe 40 by supplying the air at low or high pressure to the air transfer pipe 40 according to the changed voltage.

Here, the above-described transformer part 75 may be configured to be operated by the above-described trigger part 72 rather than the above-described voltage change manipulation part 76. In this case, the transformer part 75 is operated at level 1, which is the lowest voltage, when the trigger part 72 is manipulated one time, is operated at level 2, which is the intermediate voltage, when the trigger part 72 is manipulated two times, is operated at level 3, which is the highest voltage, when the trigger part 72 is manipulated three times, and is stopped operating when the trigger part 72 is manipulated four times. At this time, the air supply part 50 adjusts the number of revolutions of the motor or pump according to the voltage changed by the transformer part 75. The air supply part 50 is operated by changing from a first-stage mode to a third-stage mode as the intensity of the wind pressure discharged is adjusted by the adjustment of the number of revolutions, and is stopped operating when the trigger part 72 is manipulated for the fourth time. Then, the voltage change display part 77 turns on the LEDs in the number or color corresponding to this mode, and is stopped operating when the trigger part 72 is manipulated for the fourth time.

Meanwhile, the above-described transformer part 75 applies a set constant voltage corresponding to the vibrator of the above-described vibration generation part 21 so that the vibration generation part 21 is operated consistently regardless of the number of times the trigger part 72 is manipulated. The transformer part 75 applies a constant voltage to the vibrator when the trigger part 72 is operated one time for the first time, and is stopped operating and ends the voltage application when the trigger part 72 is manipulated for the fourth time. However, like the air supply part 50 described above, the transformer part 75 may be configured to apply changed power to the vibrator step by step according to the number of times the trigger part 72 is manipulated. In this case, the vibrator is operated so that the vibration intensity gradually becomes stronger and then is stopped operating.

Although the preferred embodiment of the present disclosure has been described in detail with reference to the drawings, the scope of the present disclosure is not limited to these drawings and embodiment. Therefore, various modifications or equivalent embodiments may be included in the scope of the present disclosure. The scope of the present disclosure should be determined on the basis of the descriptions in the appended claims, and all equivalents thereof should belong to the scope of the present disclosure.

## Claims

1. A medical powder spray apparatus, comprising:
a powder accommodation part (10) accommodating powder;
a powder discharge means (20) guiding and discharging the powder accommodated in the powder accommodation part (10) out of the powder accommodation part (10);
a powder transfer pipe (30) transferring the powder discharged from the powder discharge means (20);
an air transfer pipe (40) transferring air together with the powder supplied from the powder transfer pipe (30);
an air supply part (50) supplying the air to the air transfer pipe (40);
a spray means (60) spraying the powder transferred to the air transfer pipe (40) by means of the air from the air supply part (50) to outside; and
a controller (70) controlling operation of the powder discharge means (20) or the air supply part (50) by supplying power to at least one of the powder discharge means (20) and the air supply part (50),
wherein the air supply part (50) supplies the air to the air transfer pipe (40) to push the powder transferred to the air transfer pipe (40) to the spray means (60).

2. The medical powder spray apparatus of claim 1, wherein the controller (70) comprises:
a power supply part (71) supplying the power to at least one of the powder discharge means (20) and the air supply part (50); and
a trigger part (72) switched to apply the power of the power supply part (71) to at least one of the powder discharge means (20) and the air supply part (50).

3. The medical powder spray apparatus of claim 1 or 2, wherein the powder discharge means (20) comprises:
a vibration generation part (21) generating vibration;
a vibration transmission part (22) transmitting the vibration from the vibration generation part (21) to the powder accommodation part (10); and
a powder discharge part (23) dispersing and discharging the powder in the powder accommodation part (10) while being fixed to the vibration transmission part (22).

4. The medical powder spray apparatus of claim 3, wherein the vibration transmission part (22) comprises:
a vibration pipe (22a) being in contact with the vibration generation part (21), communicating with the powder transfer pipe (30), and allowing the powder to move therethrough; and
a funnel (22b) formed at an end of the vibration pipe (22a), receiving the powder discharge part (23), and fixed in a state in communication with the powder accommodation part (10).

5. The medical powder spray apparatus of claim 4, wherein a support step (22c) into which the powder discharge part (23) is insertedly fitted and supported is formed on an inner side of the funnel (22b).

6. The medical powder spray apparatus of claim 3, 4 or 5, wherein the powder discharge part (23) is formed in the shape of a triangular pyramid, a quadrangular pyramid, or a cone, and has a plurality of holes allowing the powder to pass therethrough in a dispersed state.

7. The medical powder spray apparatus of claim 6, wherein the holes of the powder discharge part (23) are formed to gradually become larger from an upper portion to a lower portion of the powder discharge part (23).

8. The medical powder spray apparatus of any one of the preceding claims, wherein a backflow prevention part (41) preventing backflow of the powder is formed in the air transfer pipe (40).

9. The medical powder spray apparatus of claim 8, wherein the backflow prevention part (41) comprises:
a ring body (41a) installed in the air transfer pipe (40) and having a space that allows the air to pass therethrough; and
a pair of prevention barrier (41b) formed on opposite sides of the ring body (41a), respectively, and opened when the air is supplied from the air transfer pipe (40) and closed when the air is not supplied from the air transfer pipe (40).

10. The medical powder spray apparatus of claim 9, wherein each of the prevention barrier (41b) is made of a flexible rubber plate having a half-disk shape, and the prevention barrier (41b) are formed such that at least a portion of a semicircular edge of each of the prevention barrier (41b) is fixed to an inner side of the ring body (41a) and straight edges of the prevention barrier (41b) are in contact with each other while facing each other.

11. The medical powder spray apparatus of any one of the preceding claims, wherein an air flow maintenance part (42) maintaining a flow of the air at a uniform pressure is mounted in the air transfer pipe (40).

12. The medical powder spray apparatus of claim 11, wherein the air flow maintenance part (42) is configured as a screw that forms a vortex of the air moving in the air transfer pipe (40), or a pipe having a plurality of holes dispersing the air moving in the air transfer pipe (40).

13. The medical powder spray apparatus of any one of the preceding claims 2 -12, wherein the trigger part (72) comprises:
a trigger (73) manipulated with a finger; and
a switch (74) operated in response to manipulation of the trigger (73) and switched to transmit the power of the power supply part (71) to the powder discharge means (20) or the air supply part (50).

14. The medical powder spray apparatus of any one of the preceding claims 2-13, wherein the air supply part (50) is configured to adjust wind pressure of the air, so that the air supply part (50) supplies the air at low pressure in response to application of low voltage power and supplies the air at high pressure in response to application of high voltage power, and
the controller (70) comprises:
a transformer part (75) changing voltage of the power supplied from the power supply part (71) to the air supply part (50) to adjust the wind pressure of the air supplied from the air transfer pipe (40); and
a voltage change display part (77) displaying a voltage change state of the transformer part (75),
wherein the transformer part (75) is controlled in operation by any one of the trigger part (72) and a voltage change manipulation part (76) operated for voltage change.

15. The medical powder spray apparatus of claim 14, wherein the voltage change display part (77) is configured as a plurality of LEDs, wherein the LEDs are set such that a small number of LEDs are turned on at low voltage and a large number of LEDs are turned on at high voltage, and the LEDs emit different colors depending on a voltage level.
